(19) Europäisches Patentamt / European Patent Office / Office européen des brevets

(11) **EP 2 331 056 B1**

(12) **FASCICULE DE BREVET EUROPEEN**

(45) Date de publication et mention de la délivrance du brevet:
**02.09.2015 Bulletin 2015/36**

(21) Numéro de dépôt: **09740376.0**

(22) Date de dépôt: **24.07.2009**

(51) Int Cl.:
*A61K 8/42* (2006.01)          *A61K 31/198* (2006.01)
*A61P 17/00* (2006.01)          *A61Q 19/00* (2006.01)
*C07C 233/47* (2006.01)

(86) Numéro de dépôt international:
**PCT/FR2009/051490**

(87) Numéro de publication internationale:
**WO 2010/026325 (11.03.2010 Gazette 2010/10)**

(54) UTILISATION D'UN N-ACYL AMINO ACIDE CHOISI PARMI LE N-PALMITOYL ALANINE, N-PALMITOYL GLYCINE, LE N-PALMITOYL ISOLEUCINE ET LE N-COCOYL ALANINE, COMME ACTIF REGULATEUR DE LA PROPORTION DE KERATINOCYTES BASAUX DE L'EPIDERME DE LA PEAU HUMAINE EXPRIMANT LA FORME NUCLEAIRE DE LA SURVIVINE; COMPOSITION COSMETIQUE ANTI-AGE EN COMPORTANT

VERWENDUNG EINER N-ACYL-AMINOSÄURE, AUSGEWÄHLT UNTER N-PALMITOYLALANIN, N-PALMITOYLGLYCIN, N-PALMITOYLISOLEUCIN UND N-COCOYLALANIN ALS WIRKSTOFF ZUR KONTROLLE DES ANTEILS VON BASALEN KERATINOZYTEN DER MENSCHLICHEN HAUTEPIDERMIS UND EXPRESSION DER NUKLEAREN ÜBERLEBENSFORM UND DIESE UMFASSENDE KOSMETISCHE ZUSAMMENSETZUNG ZUR VERHINDERUNG DER HAUTALTERUNG

USE OF AN N-ACYL AMINO ACID SELECTED FROM AMONG N-PALMITOYL ALANINE, N-PALMITOYL GLYCINE, N-PALMITOYL ISOLEUCINE, AND N-COCOYL ALANINE AS AN ACTIVE AGENT CONTROLLING THE PROPORTION OF BASAL KERATINOCYTES OF THE HUMAN SKIN EPIDERMIS AND EXPRESSING THE NUCLEAR FORM OF SURVIVING, AND ANTI-AGING COSMETIC COMPOSITION COMPRISING SAME

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **03.09.2008 FR 0855901**

(43) Date de publication de la demande:
**15.06.2011 Bulletin 2011/24**

(73) Titulaire: **Société d'Exploitation de Produits pour les Industries Chimiques SEPPIC 75007 Paris (FR)**

(72) Inventeurs:
• **DUMONT, Sandy**
  **F-81200 Caucalieres (FR)**
• **CATTUZZATO, Laetitia**
  **F-81100 Castres (FR)**
• **STOLTZ, Corinne**
  **F-94320 Thiais (FR)**
• **CHEVROT, Nathalie**
  **F-75015 Paris (FR)**

(74) Mandataire: **Conan, Philippe Claude**
  **L'Air Liquide SA**
  **Direction de la Propriété Intellectuelle**
  **75, quai d'Orsay**
  **75321 Paris Cedex 07 (FR)**

(56) Documents cités:
  **EP-A- 0 415 598          FR-A- 2 192 795**
  **US-A1- 2007 048 396**

**Description**

La présente invention se rapporte au domaine des principes actifs cosmétiques et pharmaceutiques ainsi qu'aux compositions topiques en comportant.

L'augmentation de l'espérance de vie dans les pays développés s'est constamment accompagnée par un désir irrépressible de "rajeunissement" qui s'est accentué depuis une trentaine d'années et qui se traduit par des tentatives pour retarder sinon voir disparaître, l'apparition de signes de vieillissement de la peau tels que notamment les rides. Cette évolution comportementale s'est accompagnée par un développement de la recherche scientifique dédiée à la peau ainsi que depuis une dizaine d'années, par une croissance rapide du marché des produits cosmétiques dits "anti-âge". La présente invention s'inscrit dans ce cadre de la recherche de nouvelles molécules ou compositions qui, appliqués sur la peau produisent par leur activité propre, ont cet effet "anti-âge".

Le mécanisme du vieillissement de la peau peut être expliqué comme suit :

La peau est composée de deux tissus majoritaires : le derme et l'épiderme, plus superficiel, entre lesquels s'intercale une zone de jonction, la jonction dermo-épidermique.

- <u>Le derme</u> est un tissu conjonctif dense et fibro-élastique, dont la production et le remodelage éventuel sont essentiellement assurés par les fibroblastes et qui est constitué de la matrice extracellulaire (MEC) composée de molécules fibreuses (collagéniques et élastiques), responsables des qualités esthétiques de la peau, ainsi que de la substance fondamentale. Les collagènes représentent 98% du poids sec du derme. La substance fondamentale est composée :
- De macromolécules qui comblent les espaces entre les cellules et les fibres ;
- De glycoprotéines de structure, qui permettent la connexion des cellules à la MEC et qui sont essentiellement localisées sous la jonction dermo-épidermique ;
- De protéoglycanes, dont les glycosaminoglycanes (GAG), qui se présentent sous une forme liée à une protéine (liaison covalente), ou le biglycane.
- <u>L'épiderme</u> est essentiellement composé de kératinocytes. Ces cellules prolifèrent dans l'assise basale puis se différencient progressivement pour donner les différentes couches de l'épiderme en migrant depuis la profondeur vers la surface, où elles desquament.
- <u>La jonction dermo-épidermique</u> (JDE) est la zone de jonction entre les kératinocytes basaux de l'épiderme et le derme. Elle se caractérise par la présence d'une membrane basale, dans laquelle la MEC forme un treillis mince et solide. Elle est constituée de cinq composants majeurs, *i.e.* le collagène de type IV, les laminines, l'héparan sulfate, le nidogène (ou entactine) et la fibronectine.

Les cellules souches (CS) sont des cellules non différenciées qui se caractérisent par leur capacité à maintenir l'intégrité d'un organe ou à le reconstruire. Elles sont le plus souvent localisées dans des « niches » anatomiques, lesquelles sont associées à des propriétés structurales et fonctionnelles spécifiques.

La peau adulte contient des cellules couches qui régulent son renouvellement, son aspect de surface, voire ses modifications avec le temps (Jones et Watt, 1993). On distingue deux types de cellules souches dans la peau :

- Les cellules souches folliculaires pluripotentes, essentiellement étudiées chez l'animal, et
- Les cellules souches épidermiques, qui se caractérisent par une forte capacité à s'auto-renouveler d'une part et d'autre part, à générer des cellules filles hautement différenciées.

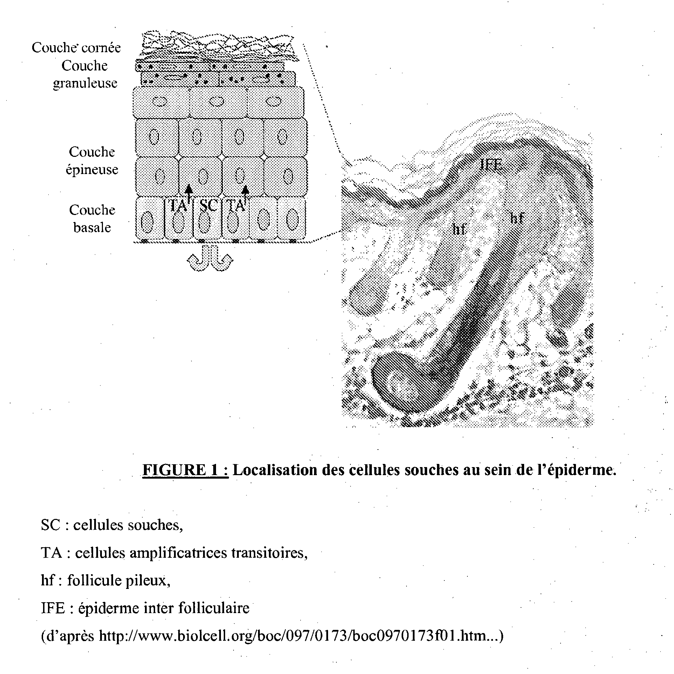

Dans l'épiderme, les CS sont situées au sein de la couche basale, leur localisation étant très spécifique (voir Figure 1). Elles représentent 1 à 10% des cellules basales (Papini et al. 2003 ; Schneider et al. 2003).

Or, sous l'effet des facteurs environnementaux, la division asymétrique des cellules souches aboutit à la génération d'une cellule fille amplificatrice transitoire (TA, 40% des cellules basales). Les cellules filles amplificatrices transitoires sont ainsi destinées à proliférer, avec un nombre de doublements de population limité, puis à devenir des cellules engagées dans un processus de différenciation (cellules post-mitotiques), au sein des couches supra-basales (Jones et Watt, 1993 ; Papini et al. 2003 ; Marconi et al. 2007). La forte capacité proliférative des cellules souches épidermiques est corrélée au fait qu'elles expriment l'intégrine beta 1 de manière plus importante que les autres kératinocytes basaux (Jones et Watt, 1993). De plus, elles présentent un pouvoir d'adhésion très important. Elles sont également fortement protégées des phénomènes d'apoptose induite par les rayonnements UVB ainsi que du processus d'anoïkis (forme spéciale d'apoptose résultant de la perte d'adhésion à la matrice extracellulaire) (Grossman et al., 2001 ; Tiberio et al., 2002 ; Marconi et al., 2004).

La survivine, appartenant à la famille des molécules anti-apoptotiques (IAP, Inhibitor of apoptosis proteins), joue un rôle prépondérant dans cette protection (Marconi et al. 2007). Initialement localisée dans le cytoplasme des cellules, cette protéine de survie doit être transloquée dans le noyau afin de permettre l'entrée de la cellule dans le cycle cellulaire (Suzuki et al. 2000). Au sein de l'épiderme, elle n'est exprimée que par certains kératinocytes basaux (Botchkareva et al. 2007 ; Marconi et al. 2007). Ainsi, les TA expriment la forme cytosolique alors que l'expression de la forme nucléaire est spécifique des cellules souches (Marconi et al. 2007).

La survivine est également exprimée par les cellules souches des follicules pileux, au sein desquels elle semble jouer un rôle dans le maintien de la croissance du follicule (Botchkareva et al. 2007). De même, elle semble être impliquée dans la protection et la survivance des cellules endothéliales, en particulier lors du processus angiogénique (O'Connor et al. 2000; Kirkiles-Smith et al. 2004).

Au cours du vieillissement, le nombre de cellules souches épidermiques est diminué et leurs fonctions sont altérées (Papini et al. 2003 ; Kwon et al., 2008).

Il existe aujourd'hui de nombreux principes actifs dits "anti-âge" ou "antirides". Plusieurs d'entre eux sont capables de réguler le phénotype et/ou les propriétés de fibroblastes humains normaux. Par exemple, de nombreux principes actifs cosmétiques stimulent la production fibroblastique de collagène. Ainsi, parmi les lipoaminoacides utilisés dans des applications cosmétiques, certains présentent des propriétés biologiques anti-âge, tels que le dipalmitoyl hydroxyproline (SEPILIFT™ DPHP).

La demande de brevet américain publiée sous le numéro US 2006/024375 divulgue que l'incorporation d'un mélange de N-palmitoyl proline, d'acide N-palmitoyl gutamique et de N-palmitoyl sarcosine dans un fond de teint induit un effet protecteur de la peau et un effet anti-âge supérieur notamment à cause de sa propriété anti-inflammatoire sur la peau et de son action comme antagoniste de la substance P.

La demande de brevet américain publiée sous le numéro US 2007/0048396 divulgue les propriétés anti-inflammatoires sur la peau d'un mélange de N-cocoyl aminoacides, parmi lesquels le N-cocoyl alanine, de sarcosine, d'aspartate de magnésium et d'aspartate de potassium.

En revanche, aucun principe actif "anti-âge" ne semble avoir été décrit pour agir sur la survivance des cellules souches épidermiques. En effet, des principes actifs tels que le polypeptide #153 utilisé dans le produit commercial AMATOKIN™, sont décrits pour augmenter le nombre de cellules souches au sein de l'épiderme en réactivant les cellules souches en dormance. Cependant, il n'existe pas de corrélation évidente entre les propriétés régulatrices des principes actifs d'une part vis à vis de l'expression de la survivine et leur efficacité anti-âge d'autre part. De même, aucun N-acyl amino acides n'a été décrit comme capable de réguler l'expression protéique de la survivine au sein des cellules souches épidermiques.

Selon un premier aspect, l'invention a pour objet l'utilisation de N-cocoyl alanine, comme actif régulateur de la proportion de kératinocytes basaux de l'épiderme de la peau humaine exprimant la forme nucléaire de la survivine.

Selon un deuxième aspect, l'invention a aussi pour objet l'utilisation de N-cocoyl alanine, pour la mise en oeuvre d'une méthode thérapeutique, destinée à maintenir la survivance de cellules souches au sein de l'épiderme de la peau humaine.

Le N-cocoyl alanine mis en oeuvre dans les utilisations telle que définies ci-dessus peut être sous forme d'acide libre ou sous forme partiellement ou totalement salifiée. Lorsqu'il est sous forme salifiée, il s'agit notamment de sels alcalins tels que les sels de sodium, de potassium ou de lithium, de sels alcalino-terreux tels que les sels de calcium, de magnésium ou de strontium ; de sel d'ammonium ou de sel d'un amino-alcool comme le sel de (2-hydroxy éthyl) ammonium. Il peut aussi s'agir de sels métalliques tels que les sels divalents de zinc ou de manganèse, les sels trivalents de fer, de lanthane, de cérium ou d'aluminium. De façon générale, le taux de salification dudit N-cocoyl alanine, dépendra en autre de son $pK_A$ et de la concentration en sels de la composition dans laquelle il est incorporé.

Par N-cocoyl alanine, on désigne un mélange de N-acyl alanine obtenu par acylation de l'alanine avec les dérivés activés d'acides gras issus de l'huile de coprah.

L'invention a aussi pour objet un procédé de traitement non thérapeutique du corps humain destiné à maintenir la survivance de cellules souches au sein de l'épiderme de la peau humaine, caractérisé en ce que l'on y applique une composition contenant un milieu cosmétiquement acceptable et une quantité efficace de N-cocoyl alanine.

Dans les utilisations et dans le procédé tels que définis ci-dessus, le N-cocoyl alanine est généralement mis en oeuvre dans une composition destinée à une application topique sur la peau et les muqueuses. Ladite composition destinée à une application topique en comprend entre 0,01 % et 10 % massique, plus particulièrement entre 0,1 % à 5 % massique, et tout particulièrement entre 1 % et 5 % massique de N-cocoyl alanine. Ledit traitement ou les dites utilisations sont mises en oeuvre par application de ladite composition destinée à une application topique, sur la surface de la peau ou de la muqueuse à traiter.

Selon un autre aspect, la présente invention a pour objet une composition cosmétique "anti-âge" caractérisée en ce qu'elle comprend de 0,01 % à 10 % massique, plus particulièrement de 0,1 % à 5 % massique et tout particulièrement de 1 % à 5 % massique de N-cocoyl alanine.

La composition mises en oeuvre dans ledit procédé ou dans lesdites utilisations, se présentent généralement sous forme de solutions aqueuses ou hydro-alcooliques diluées, sous forme d'émulsions simples ou multiples, telles que les émulsions eau dans huile (E/H), huile dans eau (H/E) ou eau dans huile dans eau (E/H/E), dans lesquelles l'huile est de nature végétale ou minérale, ou sous forme de poudre. Elles peuvent aussi être dispersées ou imprégnées sur du textile ou sur des matériaux non tissés qu'il s'agisse de lingettes, de serviettes en papier ou de vêtements.

Les compositions mises en oeuvre dans lesdits traitements se présentent généralement sous forme de solutions aqueuses ou hydro-alcooliques diluées, sous forme d'émulsions simples ou multiples, telles que les émulsions eau dans huile (E/H), huile dans eau (H/E) ou eau dans huile dans eau (E/H/E), dans lesquelles l'huile est de nature végétale ou minérale, ou sous forme de poudre. Elles peuvent aussi être dispersées ou imprégnées sur du textile ou sur des matériaux non tissés qu'il s'agisse de lingettes, de serviettes en papier ou de vêtements.

Les compositions mises en oeuvre dans lesdits traitements sont administrées au sujet sous les formes classiques utilisées en cosmétique et en pharmacie ; il s'agit plus particulièrement des administrations topique, orale ou parentérale.

De façon générale, le N-cocoyl alanine peut être associé à de nombreux types d'adjuvants ou de principes actifs utilisés dans les formulations cosmétiques, qu'il s'agisse de corps gras, de solvants organiques, d'épaississants, de gélifiants, d'adoucissants, d'antioxydants, d'opacifiants, de stabilisants, de tensioactifs moussants et/ou détergents, d'émollients, de surgraissants, de parfums, d'émulsionnants ioniques ou non, de charges, de séquestrants, de chélateurs, de conservateurs, d'huiles essentielles, de matières colorantes, de pigments, d'actifs hydrophiles ou lipophiles, d'humectants, comme par exemple la glycérine ou les glycols, de conservateurs, de colorants, d'actifs cosmétiques, de filtres solaires minéraux et/ou organiques, de charges minérales comme les oxydes de fer, les oxydes de titane et le talc, de charges synthétiques comme les nylons et les poly(méthacrylate de méthyle) réticulés ou non, d'élastomères siliconés, de séricites ou d'extraits de plantes ou encore de vésicules lipidiques, d'autobronzant comme la DHA et/ou l'érythrulose, les oxydants tels que l'eau oxygénée, ou bien tout autre ingrédient habituellement utilisé en cosmétique.

Comme exemples d'huiles que l'on peut associer au N-cocoyl alanine, on peut citer les paraffines, les isoparaffines, les huiles blanches minérales, les huiles végétales (provenant de fleurs, de fruits, de légumes, d'arbres, de céréales, d'oléagineux ...), les huiles animales, les huiles de synthèse, les huiles siliconées et les huiles fluorées ; et plus particulièrement :

- les huiles d'origine végétale, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes; les huiles végétales éthoxylées ;
- les huiles d'origine animale, telles que le squalène, le squalane ;
- les huiles minérales, telles que l'huile de paraffine, l'huile de vaseline et les isoparaffines ;
- les huiles synthétiques, notamment les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol ;
- les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les polyalphaoléfines, les polyoléfines comme le polyisobutène, les isoalcanes de synthèse comme l'isohexadécane, l'isododécane, les huiles perfluorées et les huiles de silicone. Parmi ces dernières, on peut plus particulièrement citer les diméthylpolysiloxanes, méthylphénylpolysiloxanes, les silicones modifiés par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiés par des groupements fluorés, des silicones cycliques et des silicones modifiés par des groupements alkyles.

Comme autre matière grasse que l'on peut associer au N-cocoyl alanine, on peut citer les alcools gras, linéaires ou ramifiés, saturés ou insaturés, les mélanges d'alcools gras, linéaires et/ou ramifiés, saturés et/ou insaturés, ou les acides gras, linéaires ou ramifiés, saturés ou insaturés, des mélanges d'acides gras linéaires ou ramifiés, saturés ou insaturés.

Parmi les polymères épaississants et/ou émulsionnant pouvant être associés au N-cocoyl alanine, il y a par exemple, les homopolymères ou copolymères de l'acide acrylique ou de dérivés de l'acide acrylique, les homopolymères ou copolymères de l'acide méthacrylique ou dérivés de l'acide méthacrylique, les homopolymères ou copolymères de l'acrylamide, les homopolymères ou copolymères de dérivés, de l'acrylamide, les homopolymères ou copolymères de l'acide acrylamidométhyl propanesulfonique, les homopolymères ou copolymères de monomères vinyliques, les homopolymères ou copolymères de chlorure de triméthylaminoéthylacrylate, les hydrocolloïdes d'origine végétale ou biosynthétique comme par exemple la gomme de xanthane, la gomme de karaya, les carraghénates, les alginates ; les silicates ; la cellulose et ses dérivés ; l'amidon et ses dérivés hydrophiles ; les polyuréthanes.

Parmi les polymères de type polyélectrolytes pouvant être mis en jeu dans la production d'une phase aqueuse gélifiée apte à être utilisée dans la préparation d'émulsions E/H, H/E, E/H/E ou H/E/H, ou d'un gel aqueux comprenant le SEPIBIO™ POTENTILLA 217, il y a par exemple, les copolymères de l'acide acrylique et de l'acide-2-méthyl-[(1-oxo-2-propényl)amino] 1-propane sulfonique (AMPS), les copolymères de l'acrylamide et de l'acide-2-méthyl-[(1-oxo-2-propényl)amino] 1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propane sulfonique, l'homopolymère de l'acide acrylique, les copolymères du chlorure d'acryloyl éthyl triméthyl ammonium et de l'acrylamide, les copolymères de l'AMPS et de la vinylpyrolidone, les copolymères de l'AMPS et du N,N Diméthyl acrylamide, les terpolymères de l'AMPS, de l'acide acrylique et du N,N Diméthyl acrylamide, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone. De tels polymères sont commercialisés respectivement sous les appellations SIMULGEL™ EG, SEPIGEL™ 305, SIMULGEL™ NS, SIMULGEL™ 800, SIMULGEL™ A, SIMULGEL™ EPG, SIMULGEL™ INS, SIMULGEL™ FL, SEPIGEL™ 501, SEPIGEL™ 502, SEPIPLUS™ 250, SEPIPLUS™ 265, SEPIPLUS™ 400, SEPINOV™ EMT 10, CARBOPOL™, ULTREZ™ 10, ACULYN™, PEMULEN™ TR1, PEMULEN™ TR2, LUVIGEL™ EM, SALCARE™ SC91, SALCARE™ SC92, SALCARE™ SC95, SALCARE™ SC96, FLOCARE™ ET100, FLOCARE™ ET58, HISPAGEL™, NOVEMER™ EC1, ARISTOFLEX™ AVC, ARISTOFLEX™ HBM, RAPITHIX™ A60, RAPITHIX™ A100, COSMEDIA SP et STABILEZE™ 06.

Parmi les cires pouvant être associées au N-cocoyl alanine, on peut citer par exemple la cire d'abeille, la cire de carnauba, la cire de candelilla, la cire d'ouricoury, la cire du Japon, la cire de fibre de liège ou de canne à sucre, les cires de paraffines, les cires de lignite, les cires microcristallines, la cire de lanoline, l'ozokérite, la cire de polyéthylène, les huiles hydrogénées, les cires de silicone, les cires végétales, les alcools gras et les acides gras solides à température ambiante, les glycérides solides à température ambiante.

Parmi les émulsionnants pouvant être associés au N-cocoyl alanine, on peut citer :

- les esters gras d'alkylpolyglycosides éventuellement alcoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ;
- les esters gras alcoxylés tels que le PEG 150 pentaérythrytyl tétrastéarate commercialisé sous l'appellation CROTHIX™ DS53, le PEG 55 propylène glycol oléate commercialisé sous l'appellation ANTIL™ 141 ;

- les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125;
- les acides gras, les acides gras éthoxylés, les esters d'acide gras et de sorbitol, les esters d'acides gras éthoxylés, les polysorbates, les esters de polyglycérol, les alcools gras éthoxylés, les esters de sucrose, les alkylpolyglycosides, les alcools gras sulfatés et phosphatés ou les mélanges d'alkylpolyglycosides et d'alcools gras décrits dans les demandes de brevet français 2 668 080, 2 734 496, 2 756 195, 2 762 317, 2 784 680, 2 784 904, 2 791 565, 2 790 977, 2 807 435, 2 804 432, 2 830 774, 2 830 445 ;
- les associations de tensioactifs émulsionnants choisis parmi les alkylpolyglycosides ;
- les associations d'alkylpolyglycosides et d'alcools gras, les esters de polyglycérols ou de polyglycols ou de polyols tels que les polyhydroxystéarates de polyglycols ou de polyglycérols mis en oeuvre dans les demandes de brevets français 2 852 257, 2 858 554, 2 820 316 et 2 852 258.

Parmi les tensioactifs moussants et/ou détergents pouvant être associés au N-cocoyl alanine, on peut citer : les tensioactifs anioniques, cationiques, amphotères ou non ioniques topiquement acceptables habituellement utilisés dans ce domaine d'activité.

Parmi les tensioactifs anioniques pouvant être associés au N-cocoyl alanine, on citera particulièrement les sels de métaux alcalins, les sels de métaux alcalino-terreux, les sels d'ammonium, les sels d'amines, les sels d'aminoalcools des composés suivants: les alkyléthers sulfates, les alkylsulfates, les alkylamidoéthersulfates, les alkylarylpolyéthersulfates, les monoglycérides sulfates, les alpha-oléfinesulfonates, les paraffines sulfonates, les alkylphosphates, les alkyléthersulfonates, les alkylsulfonates, les alkylamidesulfonates, les alkylarylsulfonates, les alkylcarboxylates, les alkylsulfosuccinates, les alkyléthersulfosuccinates, les alkylamidesulfosuccinates, les alkylsulfoacétates, les alkylsarcinates, les acyliséthionates, les N-acyltaurates, les acyllactylates. Parmi les tensioactifs anioniques, on citera également les lipoaminoacides, les lipoprotéines, les lipopeptides, les dérivés des lipoprotéines, les dérivés de protéines, les sels d'acides gras, les sels d'acides d'huile de coprah éventuellement hydrogénée.

Parmi les tensioactifs amphotères pouvant être associés au N-cocoyl alanine, on citera particulièrement les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques pouvant être associés dans les préparations cosmétiques comprenant les LAA, on citera particulièrement les dérivés d'ammoniums quaternaires.

Parmi les tensioactifs non ioniques pouvant être associés pouvant être associés au N-cocoyl alanine, on citera particulièrement les alkylpolyglycosides, les dérivés d'huile de ricin, les polysorbates, les amides de coprah, les N-alkyl amines, les oxydes d'amines.

Afin de potentialiser son activité, le N-cocoyl alanine, pourra être associé avec notamment les actifs connus pour leur action antioxydante, antiradicalaire, anti-âge, raffermissante, restructurante, stimulante, énergisante, oxygénante, anti ride, décontractante, hydratante, émolliente, antipelliculaire, antimicrobienne, séborrégulatrice, purifiante, apaisante, relaxante, décontractante, anti stress, amincissante, drainante, éclaircissante, dépigmentante ou pro-pigmentante, neuromodulatrice, immunomodulatrice ou tenseur. Comme exemples de principe actif que l'on peut associer au N-cocoyl alanine, on peut citer les composés ayant une action éclaircissante ou dépigmentante tels que par exemple l'arbutine, l'acide kojique, l'hydroquinone, l'acide ellagique, la vitamine C et ses dérivés, le magnésium ascorbyl phosphate, le SEPIWHITE™ MSH, le SEPICALM™ VG, l'ascorbyl glucoside, l'acide phytique, les acides de fruits, le rucinol ou résorcinol, l'acide azélaïque, l'acide lipoïque, le Vegewhite™, la Gatuline™, le Synerlight™, le Biowhite™, le Phytolight™, le Dermalight™, la Clariskin™, le Metaslow™, le Dermawhite™, l'Ethioline ™, le Melarest™, le Gigawhite™, l'Albatine™, le Lumiskin™, les extraits de polyphénols, les extraits de raisin, les extraits de pin, les extraits de vin, les extraits d'olives, les extraits de thé, les extraits de cacao, les extraits végétaux de la forêt amazonienne, les extraits de légumes, les extraits de fleurs, les extraits de fruits, les extraits de marc, les extraits de jus de pomme, les protéines N-acylées, les peptides N-acylés, les acides aminés N-acylés, comme par exemple le N-lauroyl proline, le N-linoléyl lysine, le N-linoléyl leucine, le N-octanoyl glycine, le N-undécylénoyl phénylalanine, le N-palmitoyl proline, les hydrolysâts partiels de protéines N-acylés, les acides aminés, les peptides, les hydrolysâts totaux de protéines, les hydrolysâts partiels de protéines, les polyols (par exemple, la glycérine ou le butylène glycol), les dérivés de lait, les structures associant un sucre et un polyol comme par exemple l'Aquaxyl™, l'urée, l'acide pyrrolidonecarboxylique ou les dérivés de cet acide, l'acide glycyrrhétinique, l'alpha-bisabolol, les sucres ou les dérivés des sucres, les polysaccharides ou leurs dérivés, les hydroxyacides par exemple l'acide lactique, les vitamines, les dérivés de vitamines comme le Rétinol, les dérivés du Rétinol, la vitamine E et ses dérivés, les minéraux, les enzymes, les co-enzymes, comme, le Coenzyme Q10 et ses dérivés, les hormones ou "hormone like", les extraits de soja par exemple, la Raffermine™, les extraits de blé par exemple la Tensine™ ou la Gliadine™, les extraits de Sésame comme par exemple le Sesaflash™, la carnitine et ses dérivés, la taurine et ses dérivés, la caféine et ses dérivés, la théine et ses dérivés, les extraits végétaux comme par exemple le Myoxinol™LS9736, les extraits végétaux, tels que les extraits riches en tanins, les extraits riches en isoflavones ou les extraits riches en terpènes, les extraits d'algues d'eau douce ou marines, les cires essentielles, les extraits bactériens, les minéraux, les lipides en général, les lipides tels que les céramides ou les phospholipides, les actifs ayant une action amincissante comme la caféine ou ses dérivés, les actifs améliorant la microcirculation cutanée comme par exemple les veinotoniques, les actifs drainants, les actifs à visée décongestionnante notamment au niveau des poches, les actifs ayant une activité antimicrobienne ou une action purifiante vis-à-vis des peaux grasses tels que le DEEPALINE™ PVB, le LIPACIDE™ UG, ou le SEPICONTROL™ A5 ou les dérivés de cuivre ou de zinc ou l'octopirox™ ou le Sensiva™ SC50, les actifs ayant une propriété énergisante ou stimulante comme par exemple le SEPITONIC™ M3 ou le Physiogényl™, les actifs favorisant l'oxygénation, les actifs favorisant le métabolisme des mitochondries, le panthénol et ses dérivés comme le SEPICAP™ MP, les actifs anti-age comme le SEPILIFT™ DPHP, la DEEPALINE™ PVB, le SEPIVINOL™, le SEPIVITAL™, le TIMECODE™, les actifs hydratants comme le SEPICALM™ S, le SEPICALM™ VG et le SEPILIFT™ DPHP, l'AQUAXYL™, le PROTEOL™ SAV 50, les actifs anti-âge, les actifs présentant une action de tenseur ou de lissage immédiat sur la peau comme le SESAFLASH™, les actifs "anti-photo vieillissement ", les actifs protecteurs de l'intégrité de la jonction dermo-épidermique, les actifs augmentant la synthèse des composants de la matrice extracellulaire, (comme par exemple le collagène, les élastines, les glycosaminoglycanes ...), les actifs à visée restructurante, les actifs à visée cicatrisante, les actifs à visée raffermissante, les actifs à visée « botox like », les actifs à visée « surgery like », les actifs à visée « laser like », les actifs agissant sur les rides d'expression, les actifs agissant sur les canaux calciques, les actifs présentant une action neuromodulatrice bénéfique sur le vieillissement, les actifs améliorant l'intégrité de la barrière cutanée , les actifs agissant sur des enzymes cutanées spécifiques, les actifs agissant sur des récepteurs cellulaires spécifiques, les actifs agissant sur le métabolisme des mitochondries, les actifs agissant sur le champ magnétique cutané, les actifs protecteurs des ondes magnétiques, les actifs améliorant la communication cellulaire, les actifs à visée anti-radicalaire ou anti-oxydante, les actifs à visée « tenseur », les actifs à visée antipelliculaire, anti-acnéique, apaisante, neuromodulatrice, anti-Substance P, anti-allergique, antidouleur, antistress, anti-rougeur, immunomodulatrice, tenseur, les actifs agissant sur les cellules souches comme par exemple PHYTOCELL ™, les actifs augmentant la longévité cellulaire comme par exemple LONGEVICEL™ ou des actifs à base de polyphénols. Le N-cocoyl alanine peut être idéalement utilisé au moyen de tout type d'appareil appliqué sur la peau destiné à augmenter un bénéfice cutané.

L'activité du N-cocoyl alanine, sera idéalement augmentée voire synergisée (ou potentialisée) par l'utilisation d'autres molécules ou enzymes connues pour leur action amincissante, drainante ou modulatrice de la circulation, les actifs antiradicalaires et/ou antioxydants (juste à titre d'exemple comme les superoxydes dismutases, les catalases, les peroxydases, le coenzyme Q10, les vitamines, les caroténoïdes, l'acide lipoïque, les minéraux, les polyphénols, le glutathion), les actifs favorisant l'élimination des graisses, les actifs favorisant le métabolisme de la mitochondrie, les actifs limitant l'entrée du glucose ou des acides gras dans les adipocytes, les actifs favorisant la lipolyse, les actifs inhibant la lipogénèse, les actifs inhibant la différentiation des adipocytes, les actifs agissant sur l'angiogénèse, les actifs agissant sur les canaux à glycérol, les actifs limitant le stockage des graisses et favorisant leur élimination, les actifs agissant sur l'affinement du grain de peau .... A titre d'exemple, on peut associer le N-cocoyl alanine, à l'Actisculp™, à la Biosculptine™, au Remoduline™, à l'Isoslim Complex™... , au Sveltine™ , au Slimfit™, au Sveltonyl™, à l'Unislim™, au Lipocare™, au Pleurimincyl™, au Phytotal SL™, à la Cyclolipase™, à la caféine, à la théophylline, à l'Adénosyl Mono Phosphate cyclique (AMPc), au thé vert, à la sauge, au ginko biloba, au lierre, au marron d'inde, au bambou, au ruscus, au petit houx, à la centella asiatica, à la bruyère, à l'ulmaire, au fucus, au romarin, à la saule , aux extraits de panais, aux actifs créant une sensation de « chauffe » sur la peau comme les activateurs de la microcirculation cutanée (exemple des nicotinates de méthyle, d'éthyle, d'hexyle) ou à des produits créant une sensation de « fraîcheur » sur la peau (exemple du menthol et des dérivés), aux actifs présentant une action vis à vis des cellules souches, aux actifs présentant une action sur l'épiderme, le derme, l'hypoderme et les annexes cutanés (poils, glandes sébacées, pores, ...), aux actifs présentant une action vis-à-vis de la flore cutanée.

Comme filtre solaire que l'on peut incorporer avec pouvant être associés au N-cocoyl alanine, on peut citer tous ceux figurant dans la directive cosmétique 76/768/CEE modifiée annexe VII.

Comme filtre solaire que l'on peut incorporer avec le N-cocoyl alanine, on peut citer plus particulièrement les filtres solaires lipophiles, tels que par exemple l'octorylène, l'etocrylène, l'homosalate comme l'EUSOLEX™ HMS, le paraméthoxy cinnamate d'octyle comme le PARSOL™ MCX, l'octinoxate, l'octisalate, l'avobenzone, l'oxybenzone, la benzophénone-1, la benzophénone-2, la benzophénone-3 comme l'UVINUL M-40, la benzophénone-8, la benzophénone-12, l'éthyl dihydroxypropyl PABA, le glycéryl PABA, l'éthylhexyl diméthyl PABA, l'anthranilate de menthyle, le 3-benzylidene camphor, le 4-méthylbenzylidene camphor, ou l'isopropyl dibenzoyl méthane ; les filtres solaires lipophobes tels que par exemple le dioxyde de titane, l'oxyde de zinc, l'acide phénylbenzimidazole sulfonique, la benzophénone-4, le salicylate de TEA, le PABA et le méthoxycinnamate de DEA ou encore les filtres solaires encapsulés tels que le SILASOMA™ commercialisé par la société SEIWA KAZEI ou l'UV PEARL™ commercialisé par la société MERCK.

L'écran solaire tel que défini ci-dessus peut aussi comporter un ou plusieurs absorbants d'huile tels que par exemple la silice, qu'il s'agisse de silices sphériques comme le SPHERON™ L-1500, de silice poreuse ou de silice pyrogénée, de polyméthylméthacrylate réticulés ou non réticulés comme les MICROPEARL™, les dextrines, les cyclodextrines, les tamis moléculaires comme les zéolithes, les Nylons 6 ou 12, l'aluminosilicate de sodium et de calcium, le talc ou le mica.

L'écran solaire tel que défini ci-dessus peut aussi comporter un ou plusieurs esters de l'acide néopentanoïque avec un alcool isoalkylique comportant de 10 à 22 atomes de carbone. Dans ce cas, il comprend de préférence du néopentanoate d'isodécyle, du néopentanoate d'isostéaryle ou du néopentanoate d'isoarachidyle.

Le N-cocoyl alanine, pourra être également associé à des composés présentant une propriété sensorielle, comme par exemple un effet rafraîchissant (exemple des extraits de menthe, l'alcool ...) ou des effets chauffants visant à optimiser le confort cutané.

L'étude expérimentale suivante illustre l'invention sans toutefois la limiter. Elle met en évidence que la N-cocoyl alanine, induit un effet bénéfique sur la proportion de kératinocytes exprimant la survivine. Ils permettent ainsi de maintenir la survivance des cellules souches de l'épiderme et d'exprimer une propriété "anti-âge".

**Mise en évidence de l'efficacité régulatrice du** N-cocoyl alanine selon l'invention **vis-à-vis de la proportion de kératinocytes basaux exprimant la survivine dans un** modèle *ex vivo :*

Principe :

Le modèle *ex vivo* utilisé pour mettre en évidence l'efficacité des N-acyl amino acides testés, consiste à réaliser des applications topiques de formulations cosmétiques sur des explants cutanés réalisés à partir de biopsies (déchets opératoires de chirurgie plastique) prélevées sur un donneur jeune ou sur un donneur plus âgé. Des cryo-coupes histologiques puis un immuno-marquage dirigé contre la forme nucléaire de la survivine sont ensuite réalisés afin de visualiser et quantifier la proportion de cellules positives au sein de l'épiderme. Une comparaison peut ainsi être réalisée entre le donneur jeune et le donneur âgé et/ou entre les explants traités ou non.

L'étude a été réalisée contre placebo, après 6 jours de traitement et en incluant une composition commerciale (R) de référence « anti-âge, anti-rides » du marché, le MATRIXYL™ (nom INCI : Glycerin and Water and Butylene Glycol and Carbomer and Polysorbate 20 and Palmitoyl Oligopeptide and Palmitoyl Tetrapeptide-7).

Protocole:

Le N-cocoyl alanine et la composition (R) sont formulés en gel-crème à 1% et 3% *(i.e.* dose d'utilisation conseillée), respectivement, selon le schéma de formule ci-dessous :

| Ingrédients | % |
|---|---|
| Eau | 66,8 |
| Glycérine | 5 |
| Chlorphenesine | 0,3 |
| Montanov 202 | 2 |
| Montanov 82 | 1 |
| Lanol P | 1,5 |
| Squalane végétal | 7 |
| Polyisobutène | 13 |
| Keltrol T | 0,15 |
| composé à tester | 1 |
| Montanox 60 DF | 0,5 |
| Sepiplus 400 | 0,8 |
| Sepicide LD | 0,7 |
| Parfum | 0,1 |
| TEA 50% | 0,15 |

Des explants cutanés de 8 mm de diamètre ont été préparés à partir de déchets de chirurgie plastique dégraissés. La moitié des explants (groupe PJ) provenaient de la biopsie provenant d'un patient jeune (J : 37 ans), l'autre moitié (groupe PA) de celle provenant d'un patient plus âgé (A : 52 ans). Ces explants ont été ensuite placés dans des plaques de culture 6 puits (MW6) puis maintenus à l'interface air/liquide, sans traitement, à 32°C, 5% $CO_2$ jusqu'au lendemain. Le lendemain, les formulations cosmétiques ont été appliquées à la surface d'explants du groupe "adulte" (groupe A) à l'aide d'un pinceau et à raison de 2 mg/cm$^2$. Les explants ont ensuite été cultivés à 32°C, 5% $CO_2$. Le milieu de culture a été renouvelé tous les 2 à 3 jours et les applications topiques ont été réitérées toutes les 48 heures pendant 6 jours.

Chacun des essais sont effectués en triple.

A la fin de l'incubation, les échantillons ont été lavés puis congelés dans l'isopentane refroidi à l'azote liquide avant de procéder à la réalisation des cryo-coupes histologiques. Après fixation et lavages les lames ont été placées dans une solution de saturation (lait écrémé dilué à 10% en tampon PBS *(Phosphate Buffered Saline)* Tween 20 0,05%) afin de bloquer les sites non spécifiques. Après lavages, les lames ont été incubées pendant 1 heure à température ambiante (TA) avec la dilution de l'anticorps primaire (Abcam). A la fin de l'incubation, les lames ont à nouveau été lavées et incubées pendant 1 heure à TA et à l'obscurité avec la dilution de l'anticorps secondaire couplé au fluorochrome Alexa fluor 568 (Invitrogen). Après de nouveaux lavages, une coloration nucléaire a été réalisée pendant 15 min. à TA et à l'obscurité avec la dilution du réactif Hoechst (Sigma). Les lames ont ensuite été rincées une dernière fois puis montées entre lames et lamelles à l'aide d'un milieu approprié *(Fluorescent Mounting Medium,* DakoCytomation).

Enfin, la partie épidermique des explants a été observée au microscope droit à épifluorescence (T NIKON ECLIPSE 50I) et des photographies numériques ont été réalisées (objectif x20 et/ou x40).

Analyse des résultats :

Les images ont été analysées à l'aide du logiciel LUCIA afin de déterminer la proportion relative de cellules positives pour la survivine au sein de l'épiderme.

Le pourcentage de kératinocytes positifs a été déterminé à l'aide de la formule de calcul suivante :

$$K\_Surv^+ (\%) = (\text{nombre de kératinocytes survivin }^+/\text{nb noyaux dans l'épiderme}) \times 100$$

Le pourcentage de restauration a ensuite été calculé d'après les % K_Surv$^+$ obtenus sur le groupe (PA) non traité, le groupe (PJ) non traité et le groupe A traité (groupe TA) R, Cocoyl alanine) et à l'aide de la formule de calcul suivante :

$$\text{Restauration (\%)} = \frac{(\% \text{ K\_Surv}^+ \text{ TA} - \% \text{ K\_Surv}^+ \text{ PA}) \times 100}{(\% \text{ K\_Surv}^+ \text{ PJ} - \% \text{ K\_Surv}^+ \text{ PA})}$$

Résultats :

Le tableau 1 présente les résultats obtenus avec les 3 formulations, dont celle contenant le principe actif de référence.

Tableau 1 : Effet restaurateur des principes actifs sur la proportion de kératinocytes basaux exprimant la survivine.

| | Placebo J | Placebo A | | N-cocoyl alanine |
|---|---|---|---|---|
| Variation par rapport au Placebo A (%) | 1196%* | 100% | | 1770%* |
| Restauration (%) | 100% | 0% | | 567% |
| * Résultats statistiquement significatifs par rapport au Placebo A (test de Student bilatéral à variance inégale) : p< 0,05 | | | | |

Les explants jeunes traités avec la formulation placebo (PJ) présentaient une proportion de kératinocytes exprimant la survivine (Surv$^+$) statistiquement supérieure à celle observée dans les explants plus vieux traités avec la même formulation placebo (PA). Ces résultats valident donc la pertinence du modèle en tant que modèle de vieillissement cutané.

La formulation contenant la molécule de référence n'a pas induit de modification statistiquement significative du pourcentage de kératinocytes exprimant la survivine par rapport aux explants traités avec la formulation placebo. Ce résultat est en accord avec l'absence de données relatives à l'effet de ce principe actif sur les cellules souches épidermiques.

Les formulations contenant le Cocoyl alanine ont induit une augmentation statistiquement significative du pourcentage de kératinocytes exprimant la survivine par rapport aux explants traités avec la formulation placebo. Ils présentent ainsi un effet restaurateur de 277 % et 567 %, respectivement.

Conclusion :

Le Cocoyl alanine présente un effet bénéfique sur la proportion de kératinocytes exprimant la survivine. Ils permettent ainsi de maintenir la survivance de cellules souches de l'épiderme.

**Mise en évidence "*in vivo*" de l'efficacité "antirides" du** N-cocoyl alanine **selon l'invention)**

Protocole

Les N-acyl amino acides testés ont été formulés en gel-crème à 1% dans le couple d'émulsionnants MONTANOV™ 202, MONTANOV™ 82, l'épaississant SEPIPLUS™ 400 ainsi que le MONTANOX™ 60D, afin d'effectuer des applications topiques bi-quotidiennes sur le visage de sujets caucasiens âgés de 45 ans à 76 ans et présentant des rides de vieillissement cutané (environ 20 volontaires/groupe).

La région anatomique choisie pour cette étude était la zone du contour de l'oeil (patte d'oie).

La durée du traitement a été de 42 jours.

L'absence d'irritation cutanée avait préalablement été confirmée par pose d'un patch de la formulation à tester pendant 48 heures.

Résultats

L'application répétée et biquotidienne de la formulation contenant le Cocoyl alanine a induit une diminution du nombre de rides de respectivement 40 % et 36 %, contre placebo.

**Exemples de formulation cosmétique**

Exemple 1

| Ingrédients | % |
|---|---|
| Eau | Qsp. |
| Glycérine | 5 |
| Chlorphénesine | 0,3 |
| MONTANOV™ 202 | 2 |
| MONTANOV™ 82 | 1 |
| LANOL™ P | 1,5 |
| Squalane végétal | 7 |
| Polyisobutène | 13 |
| KETROL™ T | 0,15 |
| N-cococyl alanine | 1 |
| MONTANOX™ 60 DF | 0,5 |
| SEPIPLUS™ 400 | 0,8 |
| SEPICIDE™ LD | 0,7 |
| Parfum | 0,1 |
| Tri éthanolamine à 50% dans l'eau | 0,15 |

Exemple 2

| Ingrédients | % |
|---|---|
| Eau | Qsp. |
| Glycérine | 5 |
| Chlorphénesine | 0,3 |
| MONTANOV™ 202 | 2 |
| MONTANOV™ 82 | 1 |
| LANOL™ P | 1,5 |
| Squalane végétal | 7 |
| Polyisobutène | 13 |
| KETROL™ T | 0,15 |
| N-cococyl alanine | 1 |
| N-palmitoyl glycine | 1 |
| MONTANOX™ 60 DF | 0,5 |
| SEPIPLUS™ 400 | 0,8 |
| SEPICIDE™ LD | 0,7 |
| Parfum | 0,1 |

(suite)

| Ingrédients | % |
|---|---|
| Tri éthanolamine à 50% dans l'eau | 0,15 |

Les définitions des produits commerciaux utilisés comme ingrédients sont indiquées ci- dessous :

KETROL™ T est de la gomme de xanthane commercialisée par la société KELCO.
LANOL™ P est un additif à effet stabilisant commercialisé par la société SEPPIC.
MONTANOX™ 60 DF : Stéarate de sorbitan polyéthoxylé avec 20 moles d'oxyde d'éthylène, commercialisé par la société SEPPIC;
MONTANOV™ 82 est un agent émulsionnant à base d'alcool cétéarylique et de cocoylglucoside ;
MONTANOV™ 202 (arachidyl glucoside, alcool arachidylique + alcool béhènylique), est une composition auto-émulsionnable telle que celles décrites dans WO 98/17610, commercialisée par la société SEPPIC ;
SEPICIDE™ LD : conservateur commercialisé par la société SEPPIC ;
SEPIPLUS™ 400 : Nom INCI: polyacrylate 13 / polyisobutene / polysorbate 20.

**Références bibliographiques citées dans la description**

Botchkareva et al. 2007: Botchkareva et al. J. Invest. Dermatol. (2007) Feb. 127(2): 479-82. (Epub 2006 Aug 31) "Survivin in the human hair follicle"
Grossman et al. 2001: Grossman et al. J. Clin. Invest. (2001) 108(7): 991-9 "Transgenic expression of survivin in keratinocytes côunteracts UVB-induced apoptosis and cooperates with loss of p53".
Jones et Watt, 1993: Jones et Watt, Cell. (1993) May 21;73(4):713-24 "Séparation of human epidermal stem cells from transit amplifying cells on the basis of differences in integrin function and expression".
Kirkiles-Smith et al. 2004: Kirkiles-Smith et al. J. Immunol. (2004); 172(3):1391-6 "IL-11 protects human microvascular endothelium from alloinjury in vivo by induction of survivin expression".
Kwon et al. 2008: Kwon et al. Arch. Dermatol. Res. (2008); 300(1):47-52 "Photoaging-associated changes in epidermal proliferative cell fractions in vivo".
Marconi et al. 2004: Marconi et al. J. Cell Sci. (2004) 15,117(Pt 24):5815-23 "FLICE/caspase-8 activation triggers anoikis induced by beta1-integrin blockade in human keratinocytes".
Marconi et al. 2007: Marconi et al. Stem Cells (2007) 25(1):149-55 "Survivin identifies keratinocyte stem cells and is down-regulated by anti-beta 1 integrin during anoikis".
O'Connor et al. 2000: O'Connor et al. Proc. Natl. Acad. Sci. USA (2000) 21; 97(24):13103-7 "Regulation of apoptosis at cell division by p34cdc2 phosphorylation of survivin".
Papini et al. 2003: Papini et al. Stem Cells (2003) 21(4): 481-94 "Isolation and clonal analysis of human epidermal keratinocyte stem cells in long-term culture".
Schneider et al. 2003: Schneider et al. Proc. Natl. Acad. Sci. USA (2003) 30, 100(20):11412-7 "Measuring stem cell frequency in epidermis: a quantitative in vivo functional assay for long-term repopulating cells".
Suzuki et al. 2000: Suzuki et al. Oncogene (2000) 6; 19(29):3225-34 "Survivin initiates cell cycle entry by the competitive interaction with Cdk4/p16(INK4a) and Cdk2/cyclin E complex activation".
Tiberio et al. 2002: Tiberio et al. FEBS Lett. (2002) 31, 524(1-3):139-44 "Keratinocytes enriched for stem cells are protected from anoikis via an integrin signaling pathway in a Bcl-2 dependent manner".

**Revendications**

1. Utilisation de N-cocoyl alanine, comme actif régulateur de la proportion de kératinocytes basaux de l'épiderme de la peau humaine exprimant la forme nucléaire de la survivine.

2. Utilisation de N-cocoyl alanine, pour la mise en oeuvre d'une méthode thérapeutique, destinée à maintenir la survivance de cellules souches au sein de l'épiderme de la peau humaine.

3. Procédé de traitement non thérapeutique du corps humain destiné à maintenir la survivance de cellules souches au sein de l'épiderme de la peau humaine, **caractérisé en ce que** l'on y applique une composition contenant un milieu cosmétiquement acceptable et une quantité efficace de N-cocoyl alanine.

**Patentansprüche**

1.  Verwendung von N-Cocoyl-Alanin als Wirkstoff zur Regulierung des Anteils von basalen Keratinozyten der Epidermis der menschlichen Haut, welche die nukleare Form von Survivin exprimieren.

2.  Verwendung von N-Cocoyl-Alanin für die Durchführung einer therapeutischen Methode, die dazu bestimmt ist, das Überleben von Stammzellen im Inneren der Epidermis der menschlichen Haut aufrechtzuerhalten.

3.  Verfahren zur nicht therapeutischen Behandlung des menschlichen Körpers, das dazu bestimmt ist, das Überleben von Stammzellen im Inneren der Epidermis der menschlichen Haut aufrechtzuerhalten, **dadurch gekennzeichnet, dass** eine Zusammensetzung aufgetragen wird, die ein kosmetisch verträgliches Milieu und eine wirksame Menge von N-Cocoyl-Alanin enthält.

**Claims**

1.  Use of N-cocoyl alanine as an active agent regulating the proportion of basal keratinocytes of the epidermis of human skin expressing the nuclear form of survivin.

2.  Use of N-cocoyl alanine for implementing a therapeutic method intended to maintain the survival of stem cells within the epidermis of human skin.

3.  Method for non-therapeutic treatment of the human body, intended to maintain the survival of stem cells within the epidermis of human skin, **characterised in that** a composition is applied thereto which contains a cosmetically acceptable medium and an effective quantity of N-cocoyl alanine.

Couche cornée
Couche
granuleuse

Couche
épineuse

Couche
basale

**FIGURE 1 :** Localisation des cellules souches au sein de l'épiderme.

SC : cellules souches,

TA : cellules amplificatrices transitoires,

hf : follicule pileux,

IFE : épiderme inter folliculaire

(d'après http://www.biolcell.org/boc/097/0173/boc0970173f01.htm...)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2006024375 A
- US 20070048396 A
- FR 2668080
- FR 2734496
- FR 2756195
- FR 2762317
- FR 2784680
- FR 2784904
- FR 2791565
- FR 2790977
- FR 2807435
- FR 2804432
- FR 2830774
- FR 2830445
- FR 2852257
- FR 2858554
- FR 2820316
- FR 2852258
- WO 9817610 A

**Littérature non-brevet citée dans la description**

- **BOTCHKAREVA et al.** Survivin in the human hair follicle. *J. Invest. Dermatol.,* 31 Août 2006, vol. 127 (2), 479-82
- **GROSSMAN et al.** Transgenic expression of survivin in keratinocytes côunteracts UVB-induced apoptosis and cooperates with loss of p53. *J. Clin. Invest.,* 2001, vol. 108 (7), 991-9
- **JONES ; WATT.** Séparation of human epidermal stem cells from transit amplifying cells on the basis of differences in integrin function and expression. *Cell,* 21 Mai 1993, vol. 73 (4), 713-24
- **KIRKILES-SMITH et al.** IL-11 protects human microvascular endothelium from alloinjury in vivo by induction of survivin expression. *J. Immunol.,* 2004, vol. 172 (3), 1391-6
- **KWON et al.** Photoaging-associated changes in epidermal proliferative cell fractions in vivo. *Arch. Dermatol. Res.,* 2008, vol. 300 (1), 47-52
- **MARCONI et al.** FLICE/caspase-8 activation triggers anoikis induced by beta1-integrin blockade in human keratinocytes. *J. Cell Sci.,* 2004, vol. 15 (117), 5815-23
- **MARCONI et al.** Survivin identifies keratinocyte stem cells and is down-regulated by anti-beta 1 integrin during anoikis. *Stem Cells,* 2007, vol. 25 (1), 149-55
- **O'CONNOR et al.** Regulation of apoptosis at cell division by p34cdc2 phosphorylation of survivin. *Proc. Natl. Acad. Sci. USA,* 2000, vol. 97 (24), 13103-7
- **PAPINI et al.** Isolation and clonal analysis of human epidermal keratinocyte stem cells in long-term culture. *Stem Cells,* 2003, vol. 21 (4), 481-94
- **SCHNEIDER et al.** Measuring stem cell frequency in epidermis: a quantitative in vivo functional assay for long-term repopulating cells. *Proc. Natl. Acad. Sci. USA,* 2003, vol. 100, 11412-7
- **SUZUKI et al.** Survivin initiates cell cycle entry by the competitive interaction with Cdk4/p16(INK4a) and Cdk2/cyclin E complex activation. *Oncogene,* 2000, vol. 19 (29), 3225-34
- **TIBERIO et al.** Keratinocytes enriched for stem cells are protected from anoikis via an integrin signaling pathway in a Bcl-2 dependent manner. *FEBS Lett.,* 2002, vol. 524 (1-3), 139-44